# EUROPÄISCHE PATENTSCHRIFT

(11) **EP 2 319 454 B1**
(45) Veröffentlichungstag und Bekanntmachung des Hinweises auf die Patenterteilung: **01.01.2014**
(21) Anmeldenummer: 10014333.8
(22) Anmeldetag: 05.11.2010
(51) Int. Cl.: A61F 2/06

(54) **Bypass-Vorrichtung zur Beeinflussung des Blutdrucks**
Bypass device for influencing blood pressure
Dispositif de pontage pour influencer la pression sanguine

(30) Priorität: 07.11.2009 DE 102009052349
(43) Veröffentlichungstag der Anmeldung: 11.05.2011
(73) Patentinhaber: E.S. Bio-Tech Limited, 3030 Limassol (CY)
(72) Erfinder: Doss, Mirko Dr., 60599 Frankfurt (DE)
(74) Vertreter: Müller-Gerbes Wagner Albiger Patentanwälte

(56) Entgegenhaltungen:
- WO-A1-89/01765
- WO-A1-2005/084730
- US-A1- 2004 143 319
- US-A1- 2006 217 588
- US-A1- 2007 287 879
- US-A1- 2008 234 537
- US-A1- 2009 240 277

## Beschreibung

Die Erfindung betrifft eine Bypass-Vorrichtung zur Beeinflussung des Blutdrucks, die ein Implantat mit einer Volumenkammer umfasst. Das Implantat weist Verbindungsmittel zum Verbinden der Volumenkammer an ein natürliches kardiovaskuläres System und Anpassungsmittel auf, durch die eine Volumenänderung eines Volumens der Volumenkammer bei einer Druckänderung in dem kardiovaskulären oder in der Volumenkammer ermöglicht oder bewirkt wird.

Die DE 10 2004 018 255 A1 offenbart ein Implantat in Form einer Gefäßprothese, die im Wesentlichen als Rohr mit einer elastischen Innenwandung ausgebildet ist. Durch eine Vliesstruktur wird die Dehnbarkeit der elastischen Innenwandung begrenzt, wodurch ein Druck-Dehnungsverhalten erreicht wird, das im Wesentlichen der Dehnbarkeit von natürlichen Arterien bei systolischem Druck und diastolischem Druck entspricht. Somit kann die Gefäßprothese den sogenannten Windkesseleffekt der natürlichen Arterien und insbesondere der Aorta wiederherstellen oder unterstützen. Unter der Windkesselfunktion versteht man das Zurückhalten eines Teils des vom Herzen ausgeworfenen Blutvolumens während der Systole in den elastischen zentralen Arterien und dessen kontinuierliche Abgabe während der Diastole. Die Windkesselfunktion bewirkt eine Vergleichmäßigung des arteriellen Blutflusses in der Kreislaufperipherie. Eine verminderte oder gestörte Windkesselfunktion von natürlichen Arterien bedeutet für das Herz eine grundsätzlich höhere Arbeit, wobei langfristig die Gefahr von Herzschädigungen ansteigt. Durch die elastische Innenwandung der Gefäßprothese der DE 10 2004 018 255 A1 wird durch Dehnung der Innenwandung während der Systole (Auswurfphase des Herzens) kurzzeitig eine größere Blutmenge gespeichert. Während der Diastole (Entspannung des Herzmuskels) wird das kurzzeitig gefäßprothesegespeicherte Blut aufgrund der Elastizität der Innenwandung wieder aus der Gefäßprothese herausgedrückt.

Auch aus der DE 10 2005 058 409 A1 ist ein Implantat als Ersatz eines Abschnitts der Aorta oder einer Arterie bekannt, dessen Volumen durch ein in die Gefäßwandung eingearbeitetes Federmittel elastisch verformbar ausgebildet ist. Auch dieses Implantat stützt den Windkesseleffekt.

Es sei auch auf die US 6,450,942 B1 verwiesen, die ebenfalls ein Implantat offenbart, um die Nachgiebigkeit des natürlichen arteriellen Systems wieder zu gewinnen oder zu vergrößern, um den arteriellen Blutstrom zu verbessern. Es soll dadurch die Belastung des Herzmuskels vermindert werden, da dieser wirksamer seine Arbeit verrichten kann.

Ferner offenbart die US 2007/0287879 A1 eine Federvorrichtung, die in ein Blutgefäß eingebracht wird. Durch den Federmechanismus wird das behandelte Blutgefäß zunächst elliptisch verformt, wodurch sein Volumen reduziert wird. Während der Systole wird das Gefäß dann durch den Blutdruck auf Normalgröße zurückverformt, wodurch der Blutdruck absinkt.

In der WO 89/01765 A1 ist eine prothetische Vorrichtung zur Erhöhung der Nachgiebigkeit von Arterien. Die Vorrichtung umfasst ein ballonartiges Implantat, das eine vom Blutdruck abhängige Volumenänderung zeigt.

Die US 2004/0143319 A1 zeigt ein Implantat zur Senkung von Bluthochdruck, welches parallel zu einem natürlichen Blutgefäß implantiert werden kann. Die Aufgabe des Implantats besteht hierbei darin, die natürliche Windkesselfunktion möglichst gut nachzubilden und wiederherzustellen.

Darüber hinaus seien noch die Schriften US 2008/0234537 A1 und WO 2005/084730 A1 erwähnt, die artverwandte Vorrichtungen zur Kontrolle der Durchflusskapazität des vaskulären Systems beschreiben.

In der US 2009/0240277 A1 ist des Weiteren eine Vorrichtung zur Beeinflussung des Blutdrucks offenbart. In einigen Ausführungsbeispielen ist dabei die Vorrichtung mit einer Volumenkammer versehen, die parallel zu einem Abschnitt eines natürlichen Blutgefäßes geschaltet ist und mit diesem derart verbunden ist, dass die Volumenkammer außerhalb des Blutgefäßes angeordnet ist. Die Volumenkammer ist durch ein elastisches Material begrenzt, wodurch bei Druckänderungen in der Volumenkammer ihr Volumen sich ändert.

Gleichwohl durch die bekannten Vorrichtungen aus dem Stand der Technik der natürliche Windkesseleffekt nachgebildet oder unterstützt wird und somit eine blutdrucksenkende Wirkung erzielt wird, lassen sich diese Vorrichtungen nur sehr begrenzt zur Therapie von Bluthochdruck einsetzen. Eine zusätzliche Medikamenteneinnahme durch den Patienten scheint dabei weiterhin erforderlich zu sein. Die Einnahme der Medikamente kann von teilweisen erheblichen oder schädigenden Nebenwirkungen begleitet sein, so dass stets eine Abwegung zwischen der notwendigen Blutdruckregulierung und dem Einfluss auf das Wohlbefinden des Patienten erfolgen muss. Auch ist der Erfolg dieser Therapieform weitgehend abhängig von der Mitwirkung des Patienten bei der Medikamenteneinnahme.

Der Erfindung liegt daher die Aufgabe zugrunde, eine Vorrichtung zur Beeinflussung des Blutdrucks bereitzustellen, durch die insbesondere Bluthochdruck effektiv und möglichst ohne die Notwendigkeit der Einnahme von Medikamenten therapiert werden kann.

Die der Erfindung zugrunde liegende Aufgabe wird mit der Merkmalskombination gemäß Anspruch 1 gelöst. Bevorzugte Ausführungsbeispiele können den Unteransprüchen entnommen werden.

Die Vorrichtung zur Beeinflussung des Blutdrucks gemäß Anspruch 1, insbesondere eine Bypass-Vorrichtung, zeichnet sich dadurch aus, dass eine Volumenänderung eines Volumens der Volumenkammer in einem unteren Druckbereich zwischen 50 mmHg und einen mindestens 120 mmHg betragenden Druckschwellenwert höchstens 3 cm³ und in einem oberen Druckbereich zwischen dem Druckschwellenwert und 150 mmHg mindestens 25 cm³ beträgt. Die Volumenänderung im unteren Druckbereich ist so zu verstehen, dass bei einem Druckanstieg von 50 mmHg auf den Druckschwellenwert von 100 mmHg das Volumen der Volumenkammer um höchstens 3 cm³ zunimmt. Entsprechend soll das Volumen der Volumenkammer im oberen Druckbereich bei einem Druckanstieg von 100 mmHg auf 150 mmHg mindestens um 25 cm³ zunehmen.

Dieser Sachverhalt kann vorzugsweise anhand des Verhältnisses von differentieller Volumenänderung zu differentieller Druckänderung, welches der Steigung einer Kurve in einem V-p-Diagramm entspricht, weiter konkretisiert werden. So kann die differentielle Volumenänderung pro differentieller Druckänderung für Druckwerte in einem unteren Druckbereich zwischen 50 mmHg und einem mindestens 100 mmHg betragenden Druckschwellenwert höchstens 0,2 cm³/mmHg und in einem oberen Druckbereich zwischen dem Druckschwellenwert und 150 mmHg mindestens 0,2 cm³/mmHg betragen.

Zudem wird, unabhängig von Anspruch 1, hiermit eine Bypass-Vorrichtung zur Beeinflussung des Blutdrucks offenbart, umfassend ein Implantat mit einer Volumenkammer, mit Verbindungsmitteln zum Verbinden der Volumenkammer an ein natürliches kardiovaskuläres System und mit Anpassungsmitteln, durch die eine Volumenänderung eines Volumens der Volumenkammer bei einer Druckänderung in dem kardiovaskulären System oder in der Volumenkammer ermöglicht oder bewirkt wird, wobei die differentielle Volumenänderung pro differentieller Druckänderung für Druckwerte in einem unteren Druckbereich zwischen 50 mmHg und einem mindestens 100 mmHg betragenden Druckschwellenwert höchstens 0,2 cm³/mmHg und in einem oberen Druckbereich zwischen dem Druckschwellenwert und 150 mmHg mindestens 0,2 cm³/mmHg beträgt.

Erfindungsgemäß beträgt der Druckschwellenwert mindestens 120 mmHg. Ebenfalls gute Werte bei der Therapie von Bluthochdruck lassen sich erzielen, wenn der Druckschwellenwert über 130 mmHg oder gar 140 mmHg liegt.

Für die arterielle Druckmessung ermittelt man üblicherweise zwei Werte: den oberen oder ersten Wert nennt man systolischen arteriellen Druck. Er charakterisiert den Druck im Herzen in dem Moment, in dem sich der Herzmuskel maximal zusammenzieht. Sobald sich der Herzmuskel entspannt, sinkt der arterielle Druck auf den zweiten oder unteren Druck ab (diastolischer arterieller Druck). Ein arterieller Bluthochdruck liegt vor, wenn bei wiederholter Messung ein Wert von 140 mmHg für den ersten Wert und 90 mmHg für den zweiten Wert erreicht werden.

Durch die erfindungsgemäße Bypass-Vorrichtung mit dem Implantat, die eine Volumenkammer aufweist, deren Volumen sich in dem Druckbereich unterhalb des Druckschwellenwertes (unterer Druckbereich) nur sehr wenig ändert, jedoch im Druckbereich oberhalb des Druckschwellenwertes (oberer Druckbereich) sich stark ändern kann, kann gezielt ein arterieller Bluthochdruck therapiert werden.

Die erfindungsgemäße Bypass-Vorrichtung, die parallel zu einem Abschnitt eines natürlichen Blutgefäßes implantiert wird, dient dabei der künstlichen Vergrößerung des Volumens des natürlichen kardiovaskulären Systems und bildet auf diese Weise einen in der Größe veränderbaren Volumenpuffer, der insbesondere bei hohen Drücken in dem System kurzzeitig eine gewisse Blutmenge aufnehmen und diese bei nachlassendem Systemdruck wieder abgeben kann.

Da im unteren Druckbereich sich nur geringe Volumenänderungen der Volumenkammer ergeben, ist auch ein möglicher Einfluss des Implantats auf den Windkesseleffekt im unteren Druckbereich gering. Im oberen Druckbereich hingegen werden große Volumenänderungen zugelassen, die über die Volumenänderungen hinausgehen können, die sich durch den natürlichen Windkesseleffekt bei diesen Druckverhältnissen einstellen würden. Im oberen Druckbereich können aufgrund der vergleichsweise großen Volumenänderungen auftretende Drücke effektiv und gezielt reduziert werden. Der Druckschwellenwert kann dabei, wie oben bereits ausgeführt, unterhalb dem systolischen arteriellen Druck von 140 mmHg liegen, bei dem man bei wiederholter Messung von einer arteriellen Hypertonie ausgeht.

Die Volumenänderung in dem unteren Druckbereich zwischen 50 mmHg und dem Druckschwellenwert liegt bei 3 cm³, so dass im unteren Druckbereich quasi keine Volumenänderung in der Volumenkammer des Implantats stattfindet.

Im oberen Druckbereich hingegen zwischen dem Druckschwellenwert und 150 mmHg beträgt die Volumenänderung deutlich höher als 10 cm³. Erfindungsgemäß liegt der untere Grenzwert bei 25 cm³.

Das Verhältnis aus differentieller Volumenänderung zu differentieller Druckänderung kann für Druckwerte im unteren Druckbereich zwischen 50 mmHg und dem Druckschwellenwert höchstens 0,1 cm³/mmHg oder sogar höchstens 0,06 cm³/mmHg betragen. Im oberen Druckbereich zwischen dem Druckschwellenwert und 150 mmHg kann die differentielle Volumenänderung pro differentieller Druckänderung mindestens 0,3 cm³/mmHg, mindestens 0,4 cm³/mmHg oder sogar mindestens 0,5 cm³/mmHg betragen.

Die Anpassungsmittel, durch die eine Volumenänderung des Volumens der Volumenkammer bei Druckänderung in dem kardiovaskulären System oder in der Volumenkammer ermöglicht oder bewirkt wird, können einen Energiespeicher aufweisen, der bei einem Druckanstieg Energie aufnimmt und bei einem Druckabfall Energie abgibt. Wird beispielsweise bei der Systole ein Druck von 140 mmHg erreicht, nehmen die Anpassungsmittel Energie auf, wodurch ein ansonsten höherer Druckanstieg vermieden wird. In der Diastole ist ein Druckabfall zu beobachten, wobei der Energiespeicher nun seine Energie abgibt und Blut aus der Volumenkammer drückt, wodurch der Druckabfall weniger stark ausfällt.

Der Energiespeicher kann ein elastisches Material aufweisen, das bevorzugt zumindest teilweise die Volumenkammer begrenzt. Bei Druckanstieg weitet sich dieses elastische Material auf, um dann bei einem Druckabfall wieder in Richtung seiner ursprünglichen Form zu relaxieren. Der Energiespeicher kann alternativ oder zusätzlich Federvorrichtungen umfassen, die der Volumenkammer eine bestimmte geometrische Form aufdrängen, welche sich bei einer Druckänderung ändert. Beispielsweise könnte das Implantat die Form eines Zylinders aufweisen, wobei die Federvorrichtungen den zylindrischen Querschnitt zu einem elliptischen Querschnitt zusammendrücken mit der Folge, dass das Volumen entsprechend reduziert wird. Bei einem Druckanstieg in dem Implantat wird gegen die Kraft der Federvorrichtungen wieder ein nahezu zylindrischer Querschnitt erreicht, wodurch sich das Volumen des Implantats entsprechend vergrößert. Bei Druckabfall wiederum drücken die Federvorrichtungen das Implantat wieder in die Form mit elliptischem Querschnitt. Das Volumen wird wieder reduziert.

Die Anpassungsmittel können mechanische und/oder elektrische Komponenten umfassen. Auch ist es denkbar, dass die Anpassungsmittel Magnete umfassen, durch die eine Volumenänderung der Volumenkammer gezielt beeinflusst werden kann.

In einem bevorzugten Ausführungsbeispiel ist der Druckschwellenwert einstellbar. Vorzugsweise ist dabei der Druckschwellenwert von außen einstellbar. So könnte beispielsweise über eine geeignete Ansteuerung des Implantats von außen der Druckschwellenwert so eingestellt werden, dass gewisse Blutdrücke des Patienten nicht überschritten werden. So lässt sich der Druckschwellenwert individuell für jeden Patienten einstellen. Auch ist es denkbar, dass der Druckschwellenwert in Abhängigkeit der zu erwarteten Belastung an das Herzkreislaufsystem eingestellt wird. So kann beispielsweise gegenüber einer sehr moderaten Belastung ein anderer Druckschwellenwert für zu erwartende hohe Belastungen eingestellt werden.

Der Begriff "von außen einstellbar" bedeutet somit, dass der Druckschwellenwert verändert werden kann, auch wenn das Implantat bereits implantiert ist. Es ist jedoch auch möglich, dass vor der Implantation über entsprechende Einstellungen direkt am Implantat der Druckschwellenwert eingestellt werden kann. Somit ließe sich ein Implantat herstellen, das dann für verschiedene Patienten und Bedürfnisse individuell vor der Implantation eingestellt werden kann.

Vorzugsweise weist das Implantat eine Steuerung mit einem Sensor auf, durch den ein Druck im kardiovaskulären System oder in der Volumenkammer erfasst werden kann. Bevorzugt kann dabei die Steuerung ein Signal bei Erreichen des Druckschwellenwertes oder eines mit dem Druckschwellenwert korrelierenden Wertes abgeben, welches an die Anpassungsmittel abgegeben werden kann. Liegt beispielsweise der durch den Sensor erfasste Druck unterhalb des Druckschwellenwertes, zeigen die Anpassungsmittel hinsichtlich der Volumenänderung ein anderes Verhalten als in dem Fall auf, in dem der erfasste Druck oberhalb des Druckschwellenwertes liegt.

Vorzugsweise ist ein äußerer Schutz gegen ein Einwachsen und/oder ein Einwachsen von natürlichem Gewebe an die Volumenkammer vorgesehen. Dadurch kann sichergestellt werden, dass die Volumenkammer und auch die Anpassungsmittel vor natürlichem Gewebe geschützt sind bzw. durch dieses nicht in ihren Funktionen beeinträchtigt wird.

Ist der Druckschwellenwert von außen einstellbar, bedarf es dafür geeignete Mittel. Diese Mittel können einen Empfänger umfassen, der berührungslos Signale eines von außen sendenden Senders empfängt. Zu dem kann die Vorrichtung auch einen Sender umfassen, um beispielsweise gemessene Werte hinsichtlich Blutdruck, Pulsfrequenz etc. auslesen zu können. Der Austausch bzw. das Senden oder Empfangen kann mit Hilfe von elektromagnetischen Wellen geschehen, zum Beispiel durch Radio Frequency Identification (RFID).

Zur Beeinflussung des venösen Blutdrucks - zusätzlich zur Beeinflussung des arteriellen Blutdrucks - kann ein Reservoir vorgesehen sein, das eine Kapazität von 100 ml bis 1000 ml, vorzugsweise 500 ml bis 750 ml aufweist. Das Reservoir kann dabei schneckenförmig und/oder spiralförmig sein. In einem bevorzugten Ausführungsbeispiel kann das durch das Reservoir aufnehmbare Blutvolumen vorbestimmbar und kalibrierbar sein, um eine Sollmenge des venösen Blutvolumens für jeden Patienten individuell einzustellen. Diese Einstellung des aufnehmbaren Blutvolumens kann von außen und möglicherweise auf ärztliche Anweisung regulierbar sein.

Anhand der in den Zeichnungen dargestellten Ausführungsbeispiele wird die Erfindung näher erläutert. Es zeigen:
- Fig. 1: Ein erstes Ausführungsbeispiel für ein erfindungsgemäßen Implantat;
- Fig. 2: Ein Verlauf eines Blutdrucks über einer Volumenänderung;
- Fig. 3: Ein zweites Ausführungsbeispiel des erfindungsgemäßen Implantats und einen Aortabogen;
- Fig. 4: Ein drittes Ausführungsbeispiel des erfindungsgemäßen Implantats;
- Fig. 5: Weitere Ausführungsbeispiele des erfindungsgemäßen Implantats;
- Fig. 6: Ein erstes Ausführungsbeispiel für ein venöses Reservoir; und
- Fig. 7: Ein zweites Ausführungsbeispiel für das venöse Reservoir.

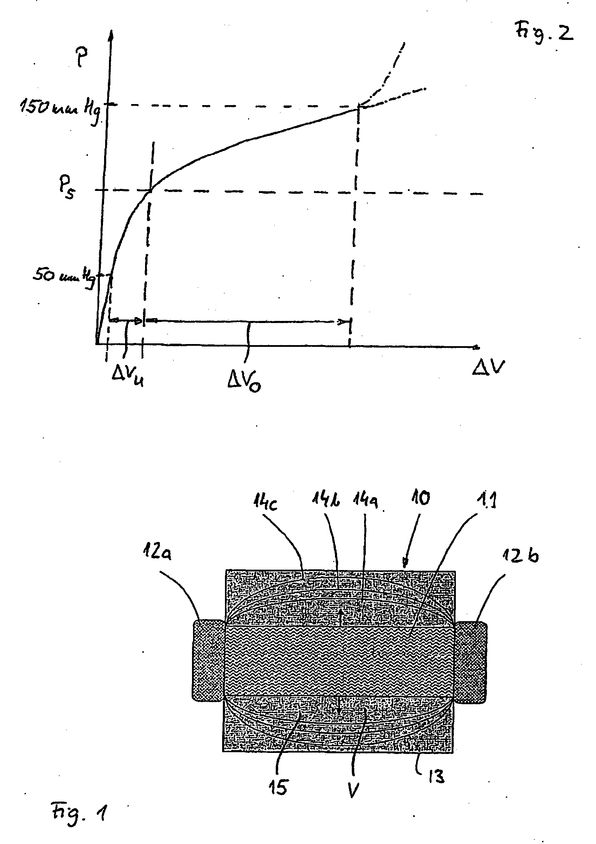

Figur 1 zeigt ein erstes Ausführungsbeispiel für ein erfindungsgemäßes Implantat, das in seiner Gesamtheit mit 10 bezeichnet wird. Das Implantat 10 umfasst ein biokompatibles elastisches Rohr 11, an dessen Enden zwei Ringe 12a, 12b befestigt sind. Die Ringe 12a, 12b dienen als Vernähungsringe, um eine Befestigung des Implantats 10 in oder an einem kardiovaskulären System zu ermöglichen. Das Implantat 10 weist zudem eine biokompatible äußere Hülle 13 auf, die verhindern soll, dass natürliches Gewebe die Funktion des elastischen Rohres oder Schlauches 11 beeinträchtigt.

Aufgrund der Elastizität des Rohres 11 weitet sich dieses aus, wenn das Implantat mit einem Druck beaufschlagt wird. Die Linien 14a, 14b und 14c sollen unterschiedliche Verformzustände des Rohres 11 darstellen, wenn in dem Rohr ein Druck herrscht, der die Rohrwandung nach außen drückt. Beim Verformungszustand 14c liegt dabei der höchste Druck an, während beim Verformungszustand 14a der kleinste Druck gegeben ist. Der Druck beim Verformungszustand 14b liegt zwischen den Drücken der Verformungszustände 14a, 14c.

Durch das verformte Rohr 11 ändert sich auch ein Volumen V einer Volumenkammer 15, die durch die Rohrwandung des Rohres 11 begrenzt wird. Im Verformzustand 14c ergibt sich somit das größte Volumen V_{c} der Volumenkammer 15 des Implantats 10.

Figur 2 zeigt den Zusammenhang zwischen einem Blutdruck P in der Volumenkammer 15 und einer Volumenänderung ΔV des Volumens der Volumenkammer 15. Durch einen Druckschwellenwert Pₛ werden ein unterer Druckbereich und ein oberer Druckbereich festgelegt. Der untere Druckbereich reicht gemäß Figur 2 von 50 mmHg bis zum dem Druckschwellenwert P_{S}, während der obere Druckbereich vom Druckschwellenwert P_{S} bis 150 mmHg reicht. Der Figur 2 kann nun entnommen werden, dass im unteren Druckbereich von 50 mmHg bis zum Druckschwellenwert P_{S} sich nur eine kleine Volumenänderung ΔV_{U} ergibt. Im oberen Druckbereich hingegen ist die Volumenänderung ΔV_{O} wesentlich größer als die Volumenänderung ΔV_{U} im unteren Druckbereich.

In dem Ausführungsbeispiel der Figur 2 liegt der Druckschwellenwert P_{S} ungefähr bei 100 mmHg. Erfingdungsgemäß nimmt der Druckschwellenwert Ps aber andere Werte an, beispielsweise 120, 130, 140 mmHg. Da bis zum Druckschwellenwert P_{S} die Volumenänderung sehr klein ist, ist auch der Einfluss des Implantats 10 auf den Blutdruck des Patienten klein. Erst ab dem Druckschwellenwert P_{S} kommt es zu einer deutlich größeren Volumenänderung ΔV_{O}, so dass dann das Implantat einen signifikanten Einfluss auf den Blutdruck des Patienten hat. Somit können Blutdrücke oberhalb des Druckschwellenwertes P_{S}, insbesondere Bluthochdruck über 140 mmHg, gezielt beeinflusst werden, ohne das bei kleinen Blutdrücken (kleiner als der Druckschwellenwert P_{S}) durch das Implantat 10 wesentlich Einfluss auf den Blutdruck genommen wird.

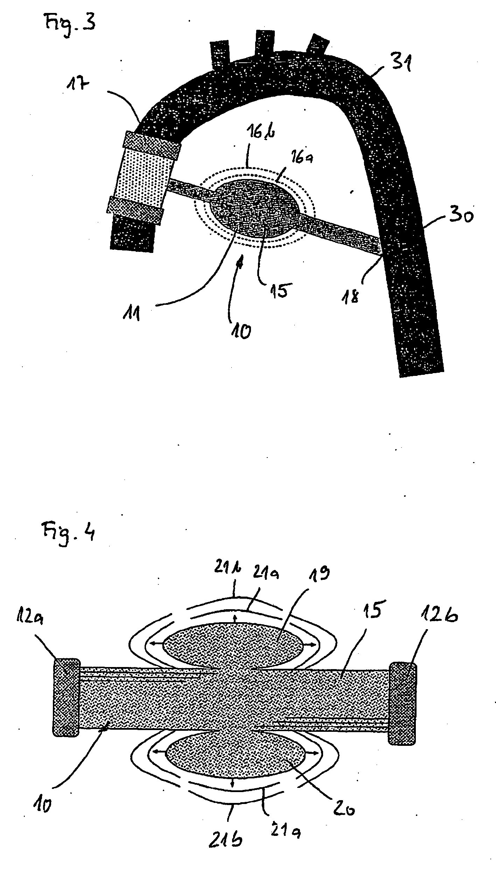

In den Figuren 3 bis 5 werden weitere Ausführungsbeispiele des erfindungsgemäßen Implantats gezeigt, wobei Teile oder Merkmale, die zu den Teilen oder Merkmalen der Figur 1 ähnlich oder identisch sind, mit gleichen Bezugszeichen versehen werden.

Figur 3 zeigt ebenfalls ein Implantat 10, welches derart mit einer Aorta 30 verbunden ist, dass das Implantat 10 zu einem oberen Abschnitt 31 der Aorta parallel geschaltet ist. Durch die gestrichelten Linien 16a, 16b ist in Figur 3 angedeutet, dass das Volumen der Volumenkammer 15 des Implantats 10 ebenfalls veränderbar ist. Das Implantat 10 der Figur 3 weist ein Vernähungselement 17 auf, das einen Teil der Aorta 30 ersetzt. An einem anderen Ende 18 des Implantats 10 ist ein hier nicht dargestellter Ring vorgesehen, um das Ende 18 des Implantats mit der Aorta 30 zu verbinden.

Figur 4 zeigt ein Ausführungsbeispiel für das Implantat 10, ebenfalls ausgestattet mit Vernähungsringen 12a, 12b. Das Implantat 10 weist hier ausdehnbare Hohlkammern 19, 20 auf, die eine entsprechende Elastizität aufweisen, um den Blutdruck in erfindungsgemäßer Weise zu beeinflussen. Auch hier wieder dienen Linien 21a, 21b der Illustration, das sich bei entsprechender Druckbeaufschlagung des Implantats 10 die Hohlkammer 19, 20 ausweiten und somit zu einer Volumenänderung der Volumenkammer 15 des Implantats führen.

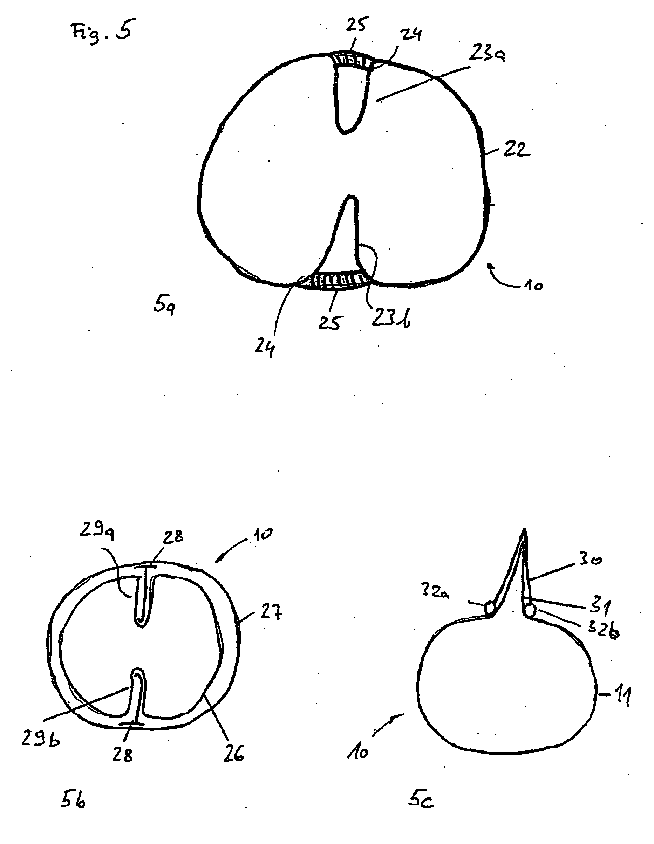

Figur 5 (Figur 5a, 5b, 5c) zeigt nun weitere Ausführungsbeispiele für das erfindungsgemäße Implantat 10, wobei hier nicht Längsschnitte, sondern schematisch Querschnitte des Implantats 10 gezeigt sind. Gemäß Ausführungsbeispiel der Figur 5a weist das Implantat 10 einen im wesentlichen kreisförmigen Schlauchquerschnitt 22 auf, der jedoch zwei nach innen gerichtete Einbauchungen 23a, 23b aufweist. An einem jeweils äußeren Ende 20 der Einbauchungen 23a, 23b ist ein Federelement 25 angeordnet, das auseinandergezogen wird, wenn das Implantat mit Druck beaufschlagt wird. Die Elastizität der Federmittel 25 in Zusammenspiel mit der Geometrie des Schlauchquerschnitts 22 ist dabei so eingestellt, dass erst ab einem bestimmten Druck die Einbauchungen gänzlich verschwinden und zu einer Ausweitung des zylindrischen Querschnitts 22 führen, so dass das Volumen des Implantats 10 deutlich ansteigt.

Das Ausführungsbeispiel der Figur 5b weist eine Innenhülle 26 sowie eine Außenhülle 27 auf. Zwischen Innenhülle 26 und Außenhülle 27 sind zwei radial nach innen gerichtete Federelemente 28 angeordnet, die zwei radial nach innen stehende Einbauchungen oder Falten 29a, 29b entstehen lassen.

Das Ausführungsbeispiel der Figur 5c zeigt eine von außen aufgesetzte Klammer 30, durch die ein verformbarer Schlauch 11 des Implantats 10 an einem Teilbereich des Umfangs des Schlauches 11 zusammengedrückt wird. Dadurch entsteht im Bereich der Klammer 30 eine Schlauchfalte 31. Bei Druckanstieg im Schlauch 11 werden die Enden 32a, 32b der Klammer auseinandergedrückt, was zu einem vergrößerten Querschnitt des Schlauches 11 und somit zu einem größeren Volumen des Implantats 10 führt.

Die Federelemente 25, 28 und 30 in Zusammenspiel mit den Einbauchungen 23a, 23b, 29a, 29b sowie Falten 31 sind so ausgelegt, dass sich eine nennenswerte Volumenänderung des Implantats 10 erst nach Erreichen des Druckschwellenwertes einstellt. Sobald dann der Druckschwellenwert erreicht ist, ergibt sich dann ein völlig neuer Zusammenhang zwischen Druckänderung und Volumenänderung.

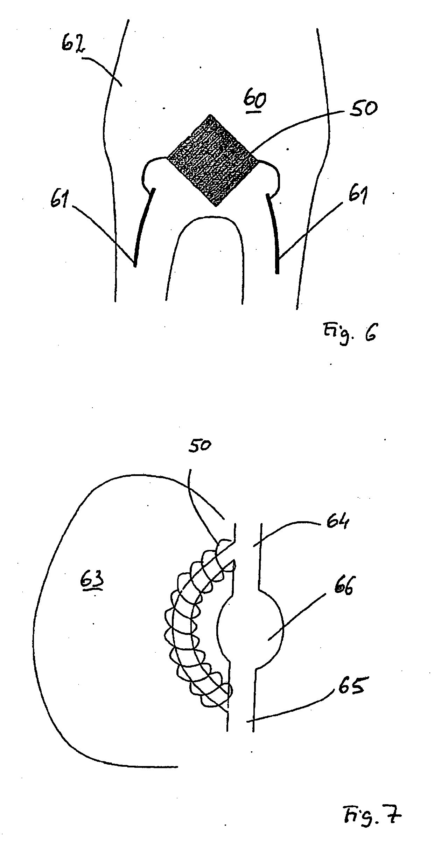

Figur 6 zeigt ein Ausführungsbeispiel für ein venöses Reservoir 50 gemäß der Erfindung. Das venöse Reservoir 16 kann eine bestimmte Menge von Blut aufnehmen. Das venöse Reservoir 50 kann, wie in Figur 6 dargestellt, in der Bauchhöhle in den femoralen Venen 61 des Patienten 62 eingesetzt sein. Das venöse Reservoir kann dabei grundsätzlich auch ohne das Implantat 10 eingesetzt werden, welches in bevorzugten Ausführungsbeispielen in den Figuren 1 bis 5 beschrieben ist. Das venöse Reservoir ist vorzugsweise dabei weniger elastisch und weniger dehnbar als das Implantat 10.

Figur 6 zeigt ein weiteres Ausführungsbeispiel des venösen Reservoirs. Das Reservoir 50 der Figur 7 ist in der Lungenhöhle 63 eingesetzt.

Das schneckenförmige oder spiralförmige Reservoir 50 dient zur Aufnahme von Blutvolumen in dem venösen System zwischen der oberen Hohlvene 64 und der unteren Hohlvene 65, so dass ein bestimmtes Blutvolumen in dem rechten Vorhof 66 bei einem gewünschten Drucklevel verschoben oder gehalten werden kann. Das Reservoir 50 ist um seine eigene Achse gewickelt, um so die externen Ausmaße zu minimieren und das interne Volumen zu maximieren.

### Bezugszeichenliste:

- 10: Implantat
- 11: Rohr/Schlauch
- 12: Vernähungsring (12a, 12b)
- 13: Hülle
- 14: Verformungszustand (14a, 14b, 14c)
- 15: Volumenkammer
- 16: Verformungszustände (16a, 16b)
- 17: Vernähungselement
- 18: anderes Ende
- 19,20: Hohlkammer
- 21: Verformungszustand (21 a, 21 b)
- 22: Querschnitt
- 23: Einbauchung (23a, 23b)
- 24: äußeres Ende
- 25: Federelement
- 26: innere Hülle
- 27: äußere Hülle
- 28: Federelement
- 29: Einbauchung (29a, 29b)
- 30: Klammer
- 31: Falte
- 32: Federende (32a, 32b)
- 50: venöses Reservoir
- 60: Bauchhöhle
- 61: femorale Vene
- 62: Patient
- 63: Lungenhöhle
- 64: obere Hohlvene
- 65: untere Hohlvene
- 66: rechter Vorhof

## Patentansprüche

1. Bypass-Vorrichtung zur Beeinflussung des Blutdrucks in einem kardiovaskulären System, umfassend ein Implantat (10) mit einer Volumenkammer (15), mit Verbindungsmitteln, die es ermöglichen, die Volumenkammer (15) zu einem Abschnitt eines natürlichen Blutgefäßes parallel zu schalten, und mit Anpassungsmitteln, durch die eine Volumenänderung eines Volumens der Volumenkammer (15) bei einer Druckänderung in dem kardiovaskulären System oder in der Volumenkammer ermöglicht oder bewirkt wird,
**dadurch gekennzeichnet, dass**
die Volumenänderung
- in einem unteren Druckbereich zwischen 50 mmHg und einem mindestens 120 mmHg betragenden Druckschwellenwert höchstens 3 cm³ und
- in einem oberen Druckbereich zwischen dem Druckschwellenwert und 150 mmHg mindestens 25 cm³
beträgt

2. Bypass-Vorrichtung nach Anspruch 1, **dadurch gekennzeichnet, dass** der Druckschwellenwert mindestens 130 mmHg beträgt.

3. Bypass-Vorrichtung nach Anspruch 2, **dadurch gekennzeichnet, dass** der Druckschwellenwert mindestens 140 mmHg beträgt.

4. Bypass-Vorrichtung nach einem der Ansprüche 1 bis 3, **dadurch gekennzeichnet, dass** der Druckschwellenwert einstellbar ist.

5. Bypass-Vorrichtung nach einem der Ansprüche 1 bis 4, **dadurch gekennzeichnet, dass** eine Steuerung mit einem Sensor vorgesehen ist, durch den ein Druck im kardiovaskulären System oder in der Volumenkammer (15) erfassbar ist.

6. Bypass-Vorrichtung nach Anspruch 5, **dadurch gekennzeichnet, dass** durch die Steuerung ein Signal bei Erreichen des Druckschwellenwerts oder eines mit dem Druckschwellenwertes korrelierenden Wertes erzeugbar ist, welches für die Mittel zur Ermöglichung der Volumenänderung bestimmt ist.

7. Bypass-Vorrichtung nach einem der Ansprüche 1 bis 6, **dadurch gekennzeichnet, dass** ein äußerer Schutz (13) gegen ein Einwachsen und/oder ein Anwachsen von natürlichem Gewebe an die Volumenkammer.

## Claims

1. A bypass device for influencing the blood pressure in a cardiovascular system, comprising an implant (10) having a volume chamber (15), with connecting means, which make it possible to connect the volume chamber (15) in parallel to a section of a natural blood vessel, and with adapting means, by means of which a volume change of the volume of the volume chamber (15) is made possible or is caused in the case of a pressure change in the cardiovascular system or in the volume chamber,
**characterised in that**
the volume change
- is at most 3 cm³ in a lower pressure range between 50 mmHg and a pressure threshold value of at least 120 mmHg, and
- is at least 25 cm³ in an upper pressure range between the pressure threshold value and 150 mmHg.

2. The bypass device according to claim 1, **characterised in that** the pressure threshold value is at least 130 mmHg.

3. The bypass device according to claim 2, **characterised in that** the pressure threshold value is at least 140 mmHg.

4. The bypass device according to any one of the claims 1 to 3, **characterised in that** the pressure threshold value is adjustable.

5. The bypass device according to any one of the claims 1 to 4, **characterised in that** a control unit with a sensor is provided, by means of which a pressure in the cardiovascular system or in the volume chamber (15) can be detected.

6. The bypass device according to claim 5, **characterised in that** a signal, which is intended for the means for making the volume change possible, can be generated by the control unit upon reaching the pressure threshold value or a value correlating with the pressure threshold value.

7. The bypass device according to any one of the claims 1 to 6, **characterised in that** an external protection (13) against natural tissue growing in and/or growing on to the volume chamber is provided.

## Revendications

1. Dispositif de pontage pour influencer la pression sanguine dans un système cardiovasculaire, comprenant un implant (10) pourvu d'une chambre volumique (15), de moyens de liaison qui permettent de monter la chambre volumique (15) en parallèle à une partie d'un vaisseau sanguin naturel et de moyens d'adaptation par lesquels un changement d'un volume de la chambre volumique (15) est rendu possible ou effectué lors d'un changement de pression dans le système cardiovasculaire ou dans la chambre volumique, **caractérisé en ce que** le changement de volume
- est d'au plus 3 cm³ dans une plage de pression inférieure entre 50 mmHg et une valeur seuil de pression d'au moins 120 mmHg, et
- est d'au moins 25 cm³ dans une plage de pression supérieure entre la valeur seuil de pression et 150 mmHg.

2. Dispositif de pontage selon la revendication 1, **caractérisé en ce que** la valeur seuil de pression est d'au moins 130 mmHg.

3. Dispositif de pontage selon la revendication 2, **caractérisé en ce que** la valeur seuil de pression est d'au moins 140 mmHg.

4. Dispositif de pontage selon une des revendications 1 à 3, **caractérisé en ce que** la valeur seuil de pression est réglable.

5. Dispositif de pontage selon une des revendications 1 à 4, **caractérisé en ce qu'**il est prévu une commande avec un capteur par lequel une pression dans le système cardiovasculaire ou dans la chambre volumique (15) peut être déterminée.

6. Dispositif de pontage selon la revendication 5, **caractérisé en ce qu'**un signal qui est destiné aux moyens servant à permettre le changement de volume peut être généré par la commande lorsque la valeur seuil de pression ou une valeur en corrélation avec la valeur seuil de pression est atteinte.

7. Dispositif de pontage selon une des revendications 1 à 6, **caractérisé par** une protection extérieure (13) contre une croissance de tissu naturel dans et/ou sur la chambre volumique.
